Europäisches Patentamt

European Patent Office

Office européen des brevets

(19)

(11) EP 1 393 670 A2

(12) EUROPEAN PATENT APPLICATION

(43) Date of publication:
03.03.2004 Bulletin 2004/10

(51) Int Cl.7: A61B 5/022, A61B 5/0285

(21) Application number: 03007545.1

(22) Date of filing: 01.04.2003

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 22.08.2002 JP 2002241701

(71) Applicant: Colin Corporation
Komaki-shi, Aichi-ken (JP)

(72) Inventors:
• Narimatsu, Kiyoyuki
Komaki-shi, Aichi-ken (JP)
• Ogura, Toshihiko
Komaki-shi, Aichi-ken (JP)
• Suzuki, Hidenori
Komaki-shi, Aichi-ken (JP)
• Honda, Takashi
Komaki-shi, Aichi-ken (JP)

(74) Representative:
Winter, Brandl, Fürniss, Hübner, Röss, Kaiser,
Polte Partnerschaft
Patent- und Rechtsanwaltskanzlei
Alois-Steinecker-Strasse 22
85354 Freising (DE)

(54) **Pulse-Wave-Propagation-Velocity-Related Information Obtaining Apparatus**

(57)  A cuff pulse wave detecting apparatus comprising: a main portion (12); a cuff (14R, 16R, 16L) which is remote from the main portion and which is adapted to be worn on a body portion of a living subject; and a pressure sensor (30, 39) which is connected to the cuff for detecting a pressure in the cuff, the cuff pulse wave detecting apparatus detecting a cuff pulse wave as a pressure oscillation transmitted from the subject to the cuff, wherein the pressure sensor is provided between the main portion and the cuff.

FIG. 3

EP 1 393 670 A2

**Description**

BACKGROUND OF THE INVENTION

Field of the Invention

The present invention relates to a cuff pulse wave detecting apparatus for detecting a cuff pulse wave as a pressure oscillation transmitted from a living subject to a cuff that is worn on the subject. Further, the present invention relates to a pulse-wave-propagation-velocity-related information obtaining apparatus for obtaining pulse-wave-propagation-velocity-related information which is related to a pulse-wave propagation velocity at which a pulse wave propagates to prescribed two body portions of a living subject, by using the cuff pulse wave detecting apparatus.

Related Art Statement

For obtaining physical information of a living subject such as pulse-wave-propagation-velocity-related information, a pulse wave detecting apparatus for detecting a pulse wave from a prescribed body portion of the subject is used. As the pulse wave detecting apparatus, there is used a cuff pulse wave detecting apparatus which includes a cuff adapted to be worn on the body portion such as an upper arm and detects a cuff pulse wave as a pressure oscillation that is transmitted from the subject to the cuff. Where the cuff pulse wave detecting apparatus is employed as the pulse wave detecting apparatus, many constituent elements or parts of the cuff pulse wave detecting apparatus can be utilized for measuring a blood pressure of the subject. Namely, the cuff pulse wave detecting apparatus also functions as a blood pressure measuring apparatus.

In the cuff pulse wave detecting apparatus, the cuff pulse wave as the pressure oscillation produced in the cuff is transmitted to a pressure sensor via a pipe connecting between the cuff and the pressure sensor, and the cuff pulse wave transmitted to the pressure sensor is converted into an electric signal. In the cuff pulse wave detecting apparatus, it takes a certain time period for the cuff pulse wave produced in the cuff to propagate via the pipe to the pressure sensor. Accordingly, the electric signal representing the cuff pulse wave is obtained the certain time period after the cuff pulse wave has been produced. In other words, there is a time lag or delay between the cuff pulse wave detected by the above-described cuff pulse wave detecting apparatus and an actual pulse wave of the subject, the time delay corresponding to the time period needed for the cuff pulse wave to propagate to the pressure sensor after it has been produced in the cuff. Further, the cuff pulse wave detected by the cuff pulse wave detecting apparatus may suffer from a phase lag or delay. Thus, the accuracy of the physical information obtained through the conventional cuff pulse wave detecting apparatus is insufficient.

SUMMARY OF THE INVENTION

It is therefore an object of the present invention to provide a cuff pulse wave detecting apparatus capable of detecting, from a living subject, a cuff pulse wave which is substantially free from the problem of time or phase delay.

The above-indicated object has been achieved by a first aspect of the present invention according to which there is provided a cuff pulse wave detecting apparatus comprising: a main portion; a cuff which is remote from the main portion and which is adapted to be worn on a body portion of a living subject; and a pressure sensor which is connected to the cuff for detecting a pressure in the cuff, the cuff pulse wave detecting apparatus detecting a cuff pulse wave as a pressure oscillation transmitted from the subject to the cuff, wherein the pressure sensor is provided between the main portion and the cuff.

In the cuff pulse wave detecting apparatus according to this aspect, a distance between the pressure sensor and the cuff is shorter than that in a conventional apparatus wherein a pressure sensor is accommodated in a main portion of the apparatus. Accordingly, the present arrangement is effective to reduce a time period required for the cuff pulse wave produced in the cuff to propagate to the pressure sensor. Therefore, the present cuff pulse wave detecting apparatus is capable of detecting, from the living subject, the cuff pulse wave that is substantially free from the problem of time or phase delay. While the pressure sensor is provided between the main portion and the cuff in the present apparatus, the pressure sensor may be provided in the cuff or may be supported by the cuff.

According to a second aspect of the present invention, there is provided a cuff pulse wave detecting apparatus comprising: a main portion; a cuff which is remote from the main portion and which is adapted to be worn on a body portion of a living subject; and a pressure sensor which is connected to the cuff via a pipe for detecting a pressure in the cuff, the cuff pulse wave detecting apparatus detecting a cuff pulse wave as a pressure oscillation transmitted from the subject to the cuff, wherein the pipe connecting between the pressure sensor and the cuff has a length that assures that a time needed for the cuff pulse wave produced in the cuff to propagate via the pipe to the pressure sensor is shorter than a shortest measurable time.

In the cuff pulse wave detecting apparatus according to the second aspect, the length of the pipe connecting between the pressure sensor and the cuff is made sufficiently short such that the time needed for the cuff pulse wave produced in the cuff to propagate via the pipe to the pressure sensor is shorter than the shortest measurable time. The shortest measurable time may correspond to the shortest operation time period of a signal processing device at which signals indicative of the pressure in the cuff and detected by the pressure sensor are read in. The shortest measurable time or the shortest operation time period will be described in greater detail in the DETAILED

DESCRIPTION OF PREFERRED EMBODIMENTS.

Therefore, the cuff pulse wave detected by the present cuff pulse wave detecting apparatus is substantially free from the problem of time or phase delay.

Preferably, the cuff pulse wave detecting apparatus, according to the above-indicated first or second aspect of the invention, is used in a pulse-wave-propagation-velocity-related information obtaining apparatus for obtaining pulse-wave-propagation-velocity-related information that is related to a pulse-wave propagation velocity at which a pulse wave propagates to two body portions of a living subject, based on two heartbeat-synchronous signals respectively detected from the two body portions. The pulse-wave-propagation-velocity-related information obtaining apparatus comprises the cuff pulse wave detecting apparatus according to the above-described first or second aspect; and wherein one of the two heartbeat-synchronous signals comprise the cuff pulse wave detected by the cuff pulse wave detecting apparatus. The present pulse-wave-propagation-velocity-related information obtaining apparatus uses, as one of the two heartbeat-synchronous signals for obtaining the pulse-wave-propagation-related information, the cuff pulse wave which is detected by the above-described cuff pulse wave detecting apparatus and which is substantially free from the problem of time delay or phase delay. Therefore, accurate pulse-wave-propagation-velocity-related information can be obtained.

Preferably, the pulse-wave-propagation-velocity-related information obtaining apparatus uses, as the two heartbeat-synchronous signals, the respective two cuff pulse waves detected by the two cuff pulse wave detecting apparatuses each according to the above-described first or second aspect of the invention. The pulse-wave-propagation-velocity-related information obtaining apparatus comprises the two cuff pulse wave detecting apparatuses, each according to the above-described first or second aspect of the invention, whose respective cuffs are adapted to be worn on the two body portions, for detecting the respective cuff pulse waves from the two body portions; and wherein the two heartbeat-synchronous signals comprise the respective cuff pulse waves detected by the two cuff pulse wave detecting apparatuses. The present pulse-wave-propagation-velocity-related information obtaining apparatus uses, as the two heartbeat-synchronous signals, the respective cuff pulse waves detected by the two cuff pulse wave detecting apparatuses constructed as described above. Therefore, more accurate pulse-wave-propagation-related information can be obtained.

BRIEF DESCRIPTION OF THE DRAWINGS

The above and optional objects, features, and advantages of the present invention will be better understood by reading the following detailed description of the preferred embodiments of the invention when considered in conjunction with the drawings, in which:

Fig. 1 is a perspective view schematically showing a construction of a pulse-wave-propagation-velocity-related information obtaining apparatus to which the present invention is applied;

Fig. 2 is a cross sectional view taken along line 2-2 of Fig. 1;

Fig. 3 is a view showing a front surface of the main body of the apparatus of Fig. 1;

Fig. 4 is a block diagram for explaining a control circuit of the apparatus of Fig. 1; and

Fig. 5 is a flow chart for explaining essential control functions of a CPU (central processing unit) of the apparatus of Fig. 1 that are needed for determining a pulse-wave propagation velocity PWV.

DETAILED DESCRIPTION OF PREFERRED EMBODIMENTS

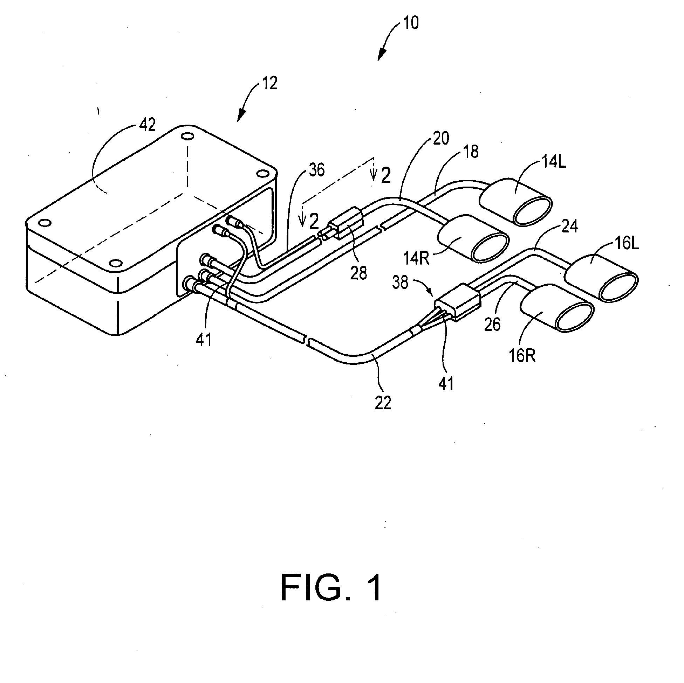

Hereinafter, there will be described an embodiment of the present invention in detail by reference to the drawings. Fig. 1 schematically shows a construction of a pulse-wave-propagation-velocity-related information obtaining apparatus 10 to which the present invention is applied. The present apparatus 10 obtains pulse-wave-propagation-related information that is related to a pulse-wave propagation velocity at which a pulse wave propagates in a living subject, and also has a function of measuring a blood pressure of the subject

The pulse-wave-propagation-velocity-related-information obtaining apparatus 10 shown in Fig. 1 includes: a box-like main body 12; a left upper-arm cuff 14L and a right upper-arm cuff 14R adapted to be worn respectively on a left and a right upper arm of the subject; and a left ankle cuff 16L and a right ankle cuff 16R adapted to be worn respectively on a left and a right ankle of the subject. The left and right upper-arm cuffs 14L, 14R are connected to the main body 12 via a pipe 18 and a pipe 20, respectively. The left and right ankle cuffs 16L, 16R are connected to the main body 12 via a pipe 24 and a pipe 26, respectively. Respective portions of the two pipes 24, 26 that are near to the main body 12 are covered with an outer sleeve 22.

A cuff-pulse-wave detecting unit 28 is provided at a position of the pipe 20 connecting between the right upper-arm cuff 14R and the main body 12, which position is remote from the right upper-arm cuff 14R by a distance of not greater than 50 cm (e.g., 50 cm or 40 cm).

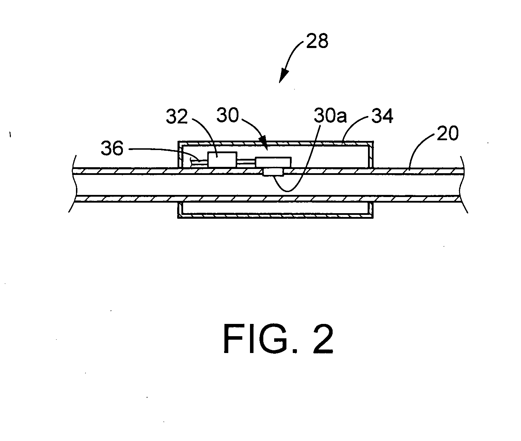

As shown in Fig. 2, the cuff-pulse-wave detecting unit 28 includes a first pressure sensor 30 having a pressure detecting portion 30a, and a pulse-wave filter circuit 32. The first pressure sensor 30 and the pulse-wave filter circuit 32 are accommodated in a protective case 34. The first pressure sensor 30 is attached to the outer circumferential surface of the pipe 20 such that the pressure detecting portion 30a projects into an inner space of the pipe 20 through its wall, so that the pressure detecting portion 30a is exposed to the inner space of the pipe 20 for detecting a first air pressure P1 in the pipe 20. The first pressure sensor 30 outputs a first pressure signal SP1 representing the detected first air pressure P1 in the pipe 20. The pulse-wave filter circuit 32 includes a band-pass filter and extracts, from the first pressure signal SP1, a first cuff-pulse-wave signal SM1 as an oscillatory component of the first air pressure P1 detected by the first pressure sensor 30.

As the first pressure sensor 30, there may be employed an electronic pressure sensor of semiconductor piezoresistance type, semiconductor capacitance type, or thin-film type.

The oscillatory component extracted from the first pressure signal SP1 by the pulse-wave filter circuit 32 represents a cuff pulse wave produced in the right upper-arm cuff 14R, i.e., a pulse wave produced from a brachial artery of the right upper arm of the subject and is propagated to the right upper-arm cuff 14R. The pulse-wave filter circuit 32 supplies the first cuff-pulse-wave signal SM1 indicative of the cuff pulse wave to the main body 12 via an electric wire (signal wire) 36. Since the first pressure signal SP1 outputted from the first pressure sensor 30 is the same as the first cuff-pulse-wave signal SM1 except that the first pressure signal SP1 contains a static-pressure component in addition to the oscillatory component, the first air pressure P1 represented by the first pressure signal SP1 may be considered as a cuff pulse wave. Since a cuff pulse wave is produced in synchronism with the heartbeat of the subject, the cuff pulse wave is a heartbeat-synchronous signal.

A cuff-pulse-wave detecting unit 38 is provided at respective positions of the pipes 24, 26, which positions are nearer to the left and right ankle cuffs 16L, 16R than the portions thereof covered with the outer sleeve 22. Described in detail, the cuff-pulse-wave detecting unit 38 is attached to the pipes 24, 26 so as to cover the pipes 24, 26, such that the detecting unit 38 is remote from the left and right ankle cuffs 16L, 16R by a distance of not greater than 50 cm. The cuff-pulse-wave detecting unit 38 includes two first pressure sensors 39 similar in construction to the first pressure sensor 30 of the cuff-pulse-wave detecting unit 28, and two pulse-wave filter circuits 40 similar in construction to the pulse-wave filter circuit 32 of the detecting unit 28. The two first pressure sensors 39 respectively detect a second air pressure P2 in the pipe 24 for the left ankle cuff 16L and a third air pressure P3 in the pipe 26 for the right ankle cuff 16R. The two pulse-wave filter circuits 40 respectively extract a second cuff-pulse-wave signal SM2 as an oscillatory component of the detected second air pressure P2, and a third cuff-pulse-wave signal SM3 as an oscillatory component of the detected third air pressure P3. The second and third cuff-pulse-wave signals SM2, SM3 represent respective cuff pulse waves.

The second and third cuff-pulse-wave signals SM2, SM3 extracted by the respective two pulse-wave-filter circuits 40 of the cuff-pulse-wave detecting unit 38 are supplied to the main body 12 via an electric wire (signal wire) 41.

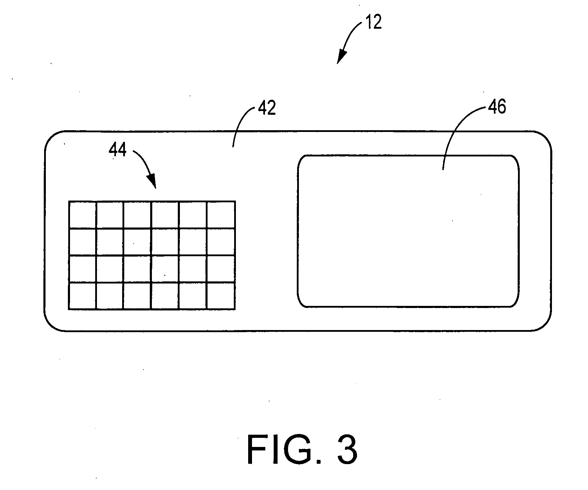

Fig. 3 shows a front surface 42 of the main body 12. On the front surface 42 of the main body 12, there are provided a plurality of input keys through which a name, an identification number, a stature, and other information of a patient are inputted, and a display device 46 which displays a measured pulse-wave propagation velocity PWV of the patient.

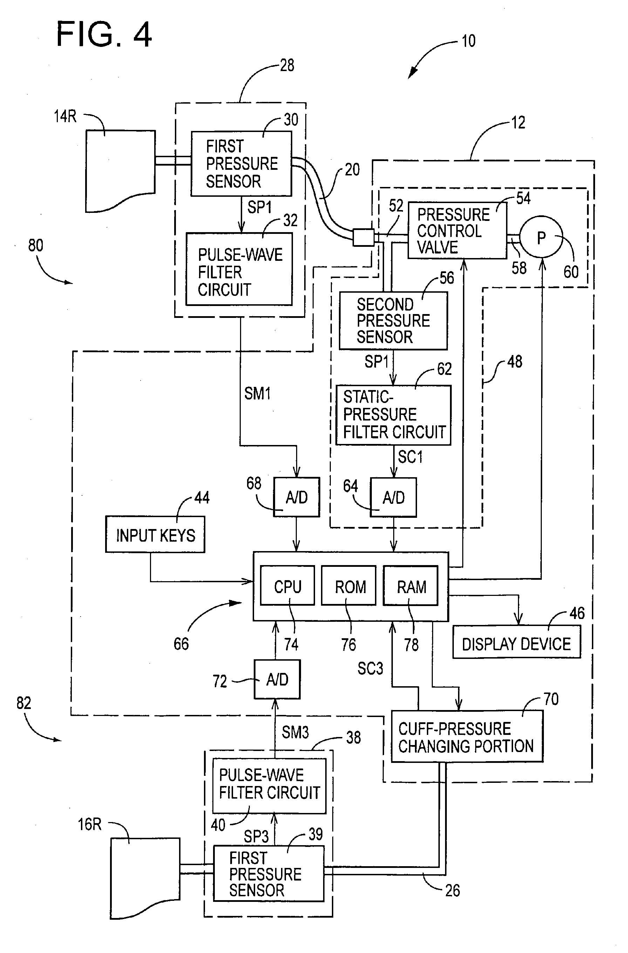

Fig. 4 is a block diagram for explaining a circuit of the present pulse-wave-propagation-velocity-related information obtaining apparatus 10. The circuit for the left ankle cuff 16L is the same as that of the right ankle cuff 16R. The circuit for changing a pressing pressure of the left upper-arm cuff 14L and detecting the cuff pulse wave produced in the left upper-arm cuff 14L is the same as that for the right upper-arm cuff 14R, except that the circuit for the left upper-arm cuff 14L includes, in place of the cuff-pulse-wave detecting unit 28, a pressure sensor corresponding to a second pressure sensor 56 of a cuff-pressure changing portion 48 (which will be described), and a static-pressure filter circuit 62 (which will be described) and a pulse-wave filter circuit 32 that are connected to the pressure sensor. In view of the above, the circuits for the left ankle cuff 16L and the left upper-arm cuff 14L are omitted from the block diagram of Fig. 4.

The cuff-pressure changing portion 48 accommodated in the main body 12 detects a pressing pressure of the right upper-arm cuff 14R (hereinafter referred to as "the first cuff pressure PC1), and changes or controls the first cuff pressure PC1. The cuff-pressure changing portion 48 includes: a pipe 52 connected to the pipe 20 for the right upper-arm cuff 14R; a pressure control valve 54 and the second pressure sensor 56 which are connected to the pipe 52; an air pump 60 connected to the pressure control valve 54 via a pipe 58; the static-pressure filter circuit 62; and an A/D (analog to digital) converter 64.

The pressure control valve 54 adjusts a pressure of a pressurized air supplied from the air pump 60, and supplies the pressure-adjusted air to the right upper-arm cuff 14R, or discharges the pressurized air from the right upper-arm cuff 14R, so as to control an air pressure in the right upper-arm cuff 14R.

Like the first pressure sensor 30 of the cuff-pulse-wave detecting unit 28, the second pressure sensor 56 detects a first air pressure P1 in the pipe 20 for the right upper-arm cuff 14R, and supplies a first pressure signal SP1 representing the detected first air pressure P1 to the static-pressure filter circuit 62.

The static-pressure filer circuit 62 has a low-pass filter and extracts, from the first pressure signal SP1, a first cuff-pressure signal SC1 representing a static-pressure component contained in the first pressure signal SP1. The first cuff-pressure signal SC1 is supplied to an electronic control device 66 via an A/D converter 64.

The first cuff-pulse-wave signal SM1 extracted from the first pressure signal SP1 by the pulse-wave filter circuit 32 is supplied to the control device 66 via an A/D converter 68. Further, the signals respectively representing the name, identification number, and stature of the patient, which signals have been inputted through the input keys 44, are supplied to the control device 66.

A cuff-pressure changing portion 70 is similar in construction to the cuff-pressure changing portion 48 described above. In the cuff-pressure changing portion 70, a pipe 52 is connected to the pipe 26 for the right ankle cuff 16R, and a static-pressure filter circuit 62 extracts a third cuff-pressure-signal SC3 representing a pressing pressure of the right ankle cuff 16R, i.e., a third cuff pressure PC3. The third cuff-pressure signal SC3 is supplied to the control device 66. The third cuff-pulse-wave signal SM3 extracted by the pulse-wave filter circuit 40 of the cuff-pulse-wave detecting unit 38 is also supplied to the control device 66 via an A/D converter 72.

The electronic control device 66 is essentially provided by a so-called microcomputer including a CPU (central processing unit) 74, a ROM (read only memory) 76, a RAM (random access memory) 78, an input-and-output (I/O) port, not shown, etc., and the CPU 74 processes signals according to control programs pre-stored in the ROM 76, while utilizing a temporary-storage function of the RAM 78. Described in detail, the control device 66 determines the first and third cuff pressures PC1, PC3 based on the first and third cuff-pressure signals SC1, SC3 respectively supplied from the static-pressure filter circuits 62, and outputs, from the I/O port, drive signals to the air pumps 60 and the pressure control valves 54 so as to control the respective operations thereof and thereby control the first and third cuff pressures PC1, PC3 to respective pre-set pulse-wave detecting pressures for measuring a pulse-wave propagation velocity PWV. The pulse-wave detecting pressures are expected to be lower than diastolic blood pressure values of the body portions of the subject around which the right upper-arm cuff 14R and the right ankle cuff 16R are respectively wound, but permit the pulse-wave signals SM1, SM3 extracted by the pulse-wave filter circuits 32, 40 to have a sufficiently great magnitude. The pulse-wave detecting pressures are pre-set at 50 mm-Hg, for instance.

As described above, the control device 66 controls the air pumps 60 and the pressure control valves 54 of the cuff-pressure changing portions 48, 70, so as to control the first and third cuff pressures PC1, PC3 to the respective pulse-wave detecting pressures, and the cuff-pulse-wave signals SM1, SM3 representing the respective cuff pulse waves are respectively detected by the pulse-wave filter circuit 32 of the cuff-pulse-wave detecting unit 28 and the pulse-wave filter circuit 40 of the cuff-pulse-wave detecting unit 38. Accordingly, the right upper-arm cuff 14R, cuff-pulse-wave detecting unit 28, cuff-pressure changing portion 48, and control device 66 cooperate with one another to provide a right-upper-arm-cuff-pulse-wave detecting apparatus 80. The right ankle cuff 16R, cuff-pulse-wave detecting unit 38, cuff-pressure changing portion 70, and control device 66 cooperate with one another to provide a right-ankle-cuff-pulse-wave detecting apparatus 82.

The CPU 74 further determines the pulse-wave propagation velocity PWV based on the cuff-pulse-wave signals SM1, SM3 respectively supplied from the pulse-wave filter circuits 32, 40 when the cuff pressures PC1, PC3 are kept at the respective pulse-wave detecting pressures. The determined pulse-wave propagation velocity PWV is displayed by the display device 46.

The cuff-pulse-wave signals SM1, SM3 supplied to the control device 66 are read in at the shortest operation time period that corresponds to a clock frequency f of the CPU 74. This shortest operation time period means the shortest measurable time. The clock frequency f is not particularly limited, and is suitably determined depending upon a speed at which the CPU 74 is required to process signals, a cost at which the CPU 74 is required to be manufactured, etc. In the present embodiment, the clock frequency f of the CPU 74 is 600 Hz, and the shortest operation time period, i.e., the shortest measurable time is 1.67 msec (millisecond).

The velocity at which the cuff pulse waves as the pressure oscillations produced in the right upper-arm cuff 14R and the left and right ankle cuffs 16L, 16R propagate in the pipes 20, 24, 26 varies depending upon the kind of the medium (i.e., the air) that transmits the pressure, the diameter and material of the pipes, etc. In the present pulse-wave-propagation-velocity-related information obtaining apparatus 10, the pulse waves propagate a distance of 1 m in from 2 ms to 3 ms. Accordingly, it takes from 1 ms to 1.5 ms or the shorter time for the cuff pulse waves to propagate from the right upper-arm cuff 14R and the left and right ankle cuffs 16L, 16R to the cuff-pulse-wave detecting units 28, 38 which are remote from the cuffs 14R, 16L, 16R by a distance of not greater than 50 cm. This time is shorter than the above-described shortest operation time period or the shortest measurable time of the CPU 74 at which the cuff-pulse-wave signals SM1, SM3 are read in. Therefore, in the present arrangement, the time needed for the cuff pulse waves produced in the right upper-arm cuff 14R and the left and right ankle cuffs 16L, 16R to propagate via the pipes 20, 24, 26 and be detected by the first pressure sensors 30, 39 of the cuff-pulse-wave detecting units 28, 38 does not cause the problem of time delay.

During a blood pressure measurement, the CPU 74 controls the air pumps 60 and the pressure control valves 54 of the cuff-pressure changing portions 40, 70, and air pumps 60 and pressure control valves 54 (both of which are not shown) for controlling the pressing pressures of the left upper-arm cuff 14L and the left ankle cuff 16L, and thereby controls the pressing pressures of the four cuffs 14L, 14R, 16L, 16R as follows: First, the pressing pressures of the four cuffs 14L, 14R, 16L, 16R are quickly increased to a pre-set target pressure. Then, the pressing pressures of the four cuffs 14L, 14R, 16L, 16R are slowly decreased at a prescribed rate. During the slow decreasing of the pressing pressures, the CPU 74 determines, according to a well-known oscillometric algorithm, blood pressure values of the superior limbs and the inferior limbs of the subject, based on the signals continuously supplied from the four static-pressure filter circuits and the four pulse-wave filter circuits.

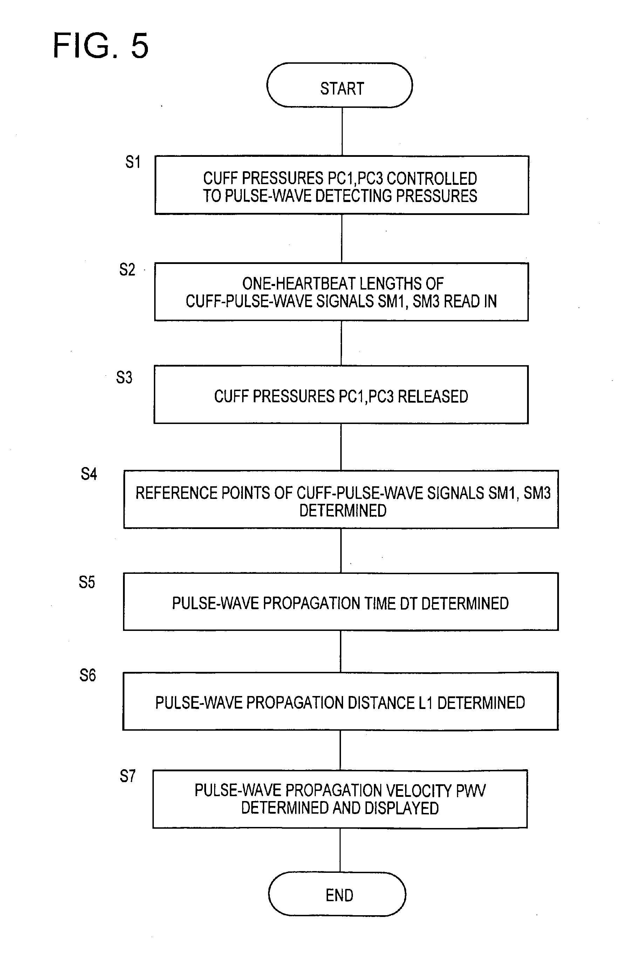

Fig. 5 is a flow chart for explaining essential control functions of the CPU 74 that are needed for determining a pulse-wave propagation velocity PWV. The present pulse-wave-propagation-velocity-related information obtaining apparatus 10 is arranged to determine a pulse-wave propagation velocity at which a pulse wave propagates to the right upper arm and the right ankle, and a pulse-wave propagation velocity at which a pulse wave propagates to the right upper arm and the left ankle. Since the respective control functions of the CPU 74 for determining the two pulse-wave propagation velocity values PWV are identical with each other, the control routine of Fig. 5 shows the control functions for determining the pulse-wave propagation velocity at which the pulse wave propagates to the right upper arm and the right ankle.

The CPU 74 carries out Step S1 (hereinafter, "Step" is omitted, if appropriate) to drive the air pumps 60 of the cuff-pressure changing portions 48, 70, and operate the pressure control valves 54 of the cuff-pressure changing portions 48, 70, so as to change the first cuff-pressure PC1 and the third cuff-pressure PC3 to the respective pre-set pulse-wave detecting pressures.

S1 is followed by S2 in which the CPU 74 reads in respective one-heartbeat lengths of the cuff-pulse-wave signals SM1, SM3 respectively supplied from the pulse-wave filter circuits 32, 40. After the signals have been read in at S2, S3 is implemented to stop the air pumps 60 and operate the pressure control valves 54 of the cuff-pressure changing portions 48, 70, so as to release the first and third cuff-pressures PC1, PC3 to an atmospheric pressure.

Subsequently, the CPU carries out S4 to determine a time of detection of a reference point of the cuff pulse wave represented by the signal SM1 and a time of detection of a corresponding reference point of the cuff pulse wave represented by the signal SM3, which signals SM1, SM3 have been read in at S2. As the reference point, a rising point or a peak point of each cuff pulse wave is employed. S4 is followed by S5 in which the CPU 74 obtains, as a pulse-wave propagation time DT, a time difference between the times of detection of the two reference points of the two cuff pulse waves.

S5 is followed by S6 to obtain, according to the following Expression (1), a propagation distance L1 as a difference between respective distances of the right upper arm and the right ankle from the heart of the subject, based on the stature T inputted through the input keys 44.

$$\text{Expression (1)} \qquad L1 = aT + b$$

(a and b are experimentally determined constants)

S6 is followed by S7 in which the CPU 74 substitutes the pulse-wave propagation time DT obtained at S5 and the pulse-wave propagation distance L1 obtained at S6, with the following Expression (2), thereby determining a pulse-wave propagation velocity PWV. In addition, the CPU 74 operates the display device 46 to display the determined pulse-wave propagation velocity PWV.

$$\text{Expression (2)} \qquad PWV = L1/DT$$

In the illustrated pulse-wave-propagation-velocity-related information obtaining apparatus 10 wherein the cuff-pulse-wave detecting units 28, 38 having the respective first pressure sensors 30, 39 are respectively provided between the main body 12 and the right upper-arm cuff 14R, and between the main body 12 and the left and right ankle cuffs 16L, 16R, the distances between the right upper-arm cuff 14R and the first pressure sensor 30 and between the left and right ankle cuffs 16L, 16R and the first pressure sensor 39 are shorter than those of a conventional arrangement wherein first pressure sensors 30, 39 are accommodated in a main body 12. Accordingly, the present arrangement effectively reduces a time required for the cuff pulse waves produced in the cuffs 14R, 16L, 16R to propagate to the first pressure sensors 30, 39, so that the obtained cuff pulse waves are substantially free from the problem of time or phase delay.

In the illustrated pulse-wave-propagation-velocity-related information obtaining apparatus 10, the length of the pipe 18 connecting between the cuff 14R and the first pressure sensor 30, the length of the pipe 24 connecting between the cuff 16L and the first pressure sensor 39, and the length of the pipe 26 connecting between the cuff 16R and the first pressure sensor 39, are made sufficiently short such that the time needed for the cuff pulse waves produced in the cuffs 14R, 16L, 16R to propagate via the pipes 18, 24, 26 to the pressure sensors 30, 39 are shorter than the shortest operation time period of the CPU 74 at which the cuff-pulse-wave signals SM1, SM3 are read in. Accordingly, the cuff pulse waves obtained by the present apparatus are substantially free from the problem of time or phase delay.

In the illustrated pulse-wave-propagation-velocity-related information obtaining apparatus 10, the cuff pulse waves substantially free from the problem of time delay are used as the two heartbeat-synchronous signals used for determining the pulse-wave propagation velocity PWV, so that the pulse-wave propagation velocity PWV can be accurately determined.

While the present invention has been described in detail in its presently preferred embodiment by reference to the drawings, the present invention may otherwise be embodied.

In the illustrated pulse-wave-propagation-velocity-related information obtaining apparatus 10, the first pressure sensor 30 is provided between the cuff 14R and the main body 12 and the first pressure sensors 39 are provided between the cuffs 16L, 16R and the main body 12. The first pressure sensor 30 may be provided in the cuff 14R or supported by the cuff 14R, and the first pressure sensors 39 may be respectively provided in the cuffs 16L, 16R, or respectively supported by the cuffs 16L, 16R.

In the illustrated pulse-wave-propagation-velocity-related information obtaining apparatus 10, the two cuff pulse waves are used as the two heartbeat-synchronous signals for determining the pulse-wave propagation velocity PWV. As one of the two heartbeat-synchronous signals, there may be used any one of a heart sound, an electrocardiographic waveform (electrocardiogram), and a photoelectric pulse wave detected by a photoelectric-pulse-wave detecting sensor adapted to be worn on, e.g., an end portion of a finger of a living subject.

Moreover, in the illustrated pulse-wave-propagation-velocity-related information obtaining apparatus 10, the pulse-wave filter circuits 32, 40 are accommodated in the cuff-pulse-wave detecting units 28, 38, respectively. The pulse-wave filter circuits 32, 40 may be accommodated in the main body 12.

The cuff pulse waves extracted by the pulse-wave filter circuits 32, 40 may be utilized to obtain physical information of the subject other than the pulse-wave-propagation-velocity-related information, such as an augmentation index, or an ejection time or period that starts with starting of ejection of blood from the left ventricle by opening of the aortic valve and ends with closing of the aortic valve.

The present invention may be embodied with various changes without departing from the spirit thereof.

**Claims**

1. A cuff pulse wave detecting apparatus comprising: a main portion (12); a cuff (14R, 16R, 16L) which is remote from the main portion and which is adapted to be worn on a body portion of a living subject; and a pressure sensor (30, 39) which is connected to the cuff for detecting a pressure in the cuff, the cuff pulse wave detecting apparatus detecting a cuff pulse wave as a pressure oscillation transmitted from the subject to the cuff, the apparatus being **characterized in that**:

   the pressure sensor is provided between the main portion and the cuff.

2. A cuff pulse wave detecting apparatus according to claim 1, wherein a distance between the pressure sensor and the cuff is not greater than 50 cm.

3. A cuff pulse wave detecting apparatus comprising: a main portion (12); a cuff (14R, 16R, 16L) which is remote from the main portion and which is adapted to be worn on a body portion of a living subject; and a pressure sensor (30, 39) which is connected to the cuff via a pipe (20, 24, 26) for detecting a pressure in the cuff, the cuff pulse wave detecting apparatus detecting a cuff pulse wave as a pressure oscillation transmitted from the subject to the cuff, the apparatus being **characterized in that**:

   the pipe connecting between the pressure sensor and the cuff has a length that assures that a time needed for the cuff pulse wave produced in the cuff to propagate via the pipe to the pressure sensor is shorter than a shortest measurable time.

4. A cuff pulse wave detecting apparatus according to claim 3, wherein the length of the pipe is not greater than 50 cm.

5. A cuff pulse wave detecting apparatus according to claim 3 or 4, wherein the pressure sensor is supported by the pipe such that a portion of the pressure sensor is exposed to an inner space of the pipe through a wall of the pipe.

6. A cuff pulse wave detecting apparatus according to any one of claims 3 through 5, wherein the pressure sensor outputs a signal representative of the pressure in the cuff, the apparatus further comprising a signal processing device (66, 74) which periodically reads in the signal outputted from the pressure sensor, at a prescribed time period that corresponds to the shortest measurable time.

7. A cuff pulse wave detecting apparatus according to claim 6, wherein the prescribed time period corresponds to a clock frequency (f) of the signal processing device.

**8.** A cuff pulse wave detecting apparatus according to claim 6 or 7, wherein the signal processing device is accommodated in the main portion.

**9.** A cuff pulse wave detecting apparatus according to any one of claims 1 through 8, wherein the pressure sensor detects the cuff pulse wave.

**10.** A pulse-wave-propagation-velocity-related information obtaining apparatus for obtaining pulse-wave-propagation-related information that is related to a pulse-wave-propagation velocity at which a pulse wave propagates to two body portions of a living subject, based on two heartbeat-synchronous signals respectively detected from the two body portions, the apparatus being **characterized by** comprising

the cuff pulse wave detecting apparatus according to any one of claims 1 through 9; and in that

the cuff pulse wave detected by the cuff pulse wave detecting apparatus is used as one of the two heartbeat-synchronous signals.

**11.** A pulse-wave-propagation-velocity-related information obtaining apparatus for obtaining pulse-wave-propagation-related information that is related to a pulse-wave-propagation velocity at which a pulse wave propagates to two body portions of a living subject, based on two heartbeat-synchronous signals respectively detected from the two body portions, the apparatus being **characterized by** comprising

the two cuff pulse wave detecting apparatuses, each according to any one of claims 1 through 9, whose respective cuffs are adapted to be worn on the two body portions, for detecting the respective cuff pulse waves from the two body portions; and in that

the respective cuff pulse waves detected by the two cuff pulse wave detecting apparatuses are used as the two heartbeat-synchronous signals.

**12.** A pulse-wave-propagation-velocity-related information obtaining apparatus according to claim 10 or 11, further comprising pulse-wave-propagation-velocity-related information obtaining means which includes pulse-wave-propagation-velocity determining means for determining the pulse-wave propagation velocity, by dividing a difference between respective distances between the two body portions and a heart of the subject by a difference between a time of detection of a reference point of one of the two heartbeat-synchronous signals and a time of detection of a corresponding reference point of the other of the two heartbeat-synchronous signals.

FIG. 1

FIG. 2

FIG. 3

# FIG. 4

# FIG. 5

```
              ┌─────────────┐
              │    START    │
              └──────┬──────┘
                     │
   S1  ┌─────────────────────────────────────────┐
       │   CUFF PRESSURES PC1,PC3 CONTROLLED      │
       │     TO PULSE-WAVE DETECTING PRESSURES    │
       └──────────────────┬──────────────────────┘
                          │
   S2  ┌─────────────────────────────────────────┐
       │         ONE-HEARTBEAT LENGTHS OF         │
       │   CUFF-PULSE-WAVE SIGNALS SM1, SM3 READ IN│
       └──────────────────┬──────────────────────┘
                          │
   S3  ┌─────────────────────────────────────────┐
       │     CUFF PRESSURES PC1,PC3 RELEASED      │
       └──────────────────┬──────────────────────┘
                          │
   S4  ┌─────────────────────────────────────────┐
       │ REFERENCE POINTS OF CUFF-PULSE-WAVE SIGNALS SM1, SM3 │
       │                DETERMINED                │
       └──────────────────┬──────────────────────┘
                          │
   S5  ┌─────────────────────────────────────────┐
       │  PULSE-WAVE PROPAGATION TIME DT DETERMINED│
       └──────────────────┬──────────────────────┘
                          │
   S6  ┌─────────────────────────────────────────┐
       │ PULSE-WAVE PROPAGATION DISTANCE L1 DETERMINED│
       └──────────────────┬──────────────────────┘
                          │
   S7  ┌─────────────────────────────────────────┐
       │    PULSE-WAVE PROPAGATION VELOCITY PWV   │
       │        DETERMINED AND DISPLAYED          │
       └──────────────────┬──────────────────────┘
                          │
              ┌─────────────┐
              │     END     │
              └─────────────┘
```